# EUROPEAN PATENT APPLICATION

(11) **EP 0 655 241 A1**
(43) Date of publication of application: **31.05.1995**
(21) Application number: 94308737.9
(22) Date of filing: 25.11.1994
(51) Int. Cl.: A61K 9/48

(54) **Soft gelatine capsule containing a mixture with a high water content**

(30) Priority: 25.11.1993 FR 9314277
(71) Applicant: R.P. SCHERER CORPORATION, Troy, Michigan 48007-7060 (US)
(72) Inventor: Altmann, Alain, F-67000 Strasbourg (FR)
(74) Representative: Hitchcock, Esmond Antony

(57) **Abstract**

A soft gelatine capsule comprises a shell containing a filling with a high water content. The filling includes at least one constituent in the form of colloidal silica or silicon dioxide, which has the effect of fixing the water in the filling, and preventing its migration to the shell and consequential breakdown of the shell.

## Description

The present invention relates to soft gelatine capsules for use in the field of predosed forms for pharmaceutical, veterinary, dietetic, food, cosmetic or other products.

The use of soft capsules in predosed forms for pharmaceutical, veterinary, dietetic, food, cosmetic or other purposes has been known for many years and is currently very widespread. These soft capsules are generally manufactured either according to a so-called drop process or according to one known as the rotary die process using rotating moulds. This process is described in the September 1985 edition of Pharmaceutical Technology, to which reference is directed.

A known rotary die process consists of forming endless strips of capsule shell material from a molten mass which is of viscous consistency and has a gelatine base, this mass being maintained at a temperature of approximately 700°C. The thickness of the gelatine strip can of course be selected according to the eventual use of the capsule. The gelatine mass comprises the gelatine itself, glycerine and water in varying proportions depending on the eventual use and the formulations to be encapsulated. Typical proportions are approximately the following: 40%, 30% and 30% of gelatine, glycerine and water respectively. The molten mass may be provided with additives.

The gelatine strips are guided between two moulds rotating in opposite directions, between which a device is inserted for injection of the product to be encapsulated, commonly known as a segment. The two moulds between which the capsules are formed each have cavities in the shape of a capsule half.

When the edges of the two opposite cells join together by the rotation of the moulds, the capsule filling, which normally consists of a mixture of various ingredients, is injected between the two films of gelatine by means of the injection segment which is fed by pumps with a very accurate delivery, thereby giving each capsule its appropriate volume of filling. During this operation, the injection segment is heated slightly such that the surfaces of the gelatine films become softened, which allows the two halves of the capsule to be formed and weld together. The contents of the capsule are not affected by the heating.

After they have been filled, the capsules are ejected from the cylinder by a series of ejectors fitted in the cavities, and are then conveyed to a pre-drying system. In a succession of drying phases, the capsules are hardened until they have a final water content in the gelatine envelope that permits them to be handled for wrapping and packaging purposes. An acceptable water content in this context is normally in the range of around 6% to 10% by weight.

At present, due to the nature of the gelatine envelope itself, which is a hydrosoluble compound, the contents or filling of the capsules is prepared using a solution, suspension or emulsion, with a vegetable or mineral oil base, such as for example silicones, or a hydrophilic excipient base such as, for example, polyethylene glycol. A filling in the form of an aqueous preparation would result in the capsule envelope breaking down, leading initially to deformation. In time such a phenomenon can cause agglomeration between packaged capsules, or even rupture of capsules and thus the escape of the contents or filling. We have noted capsule breakdown when the water content of the filling is at least 10% by weight. Furthermore, the presence of water in emulsions does not allow durable stability of these to be achieved.

We have found that fillings with high water contents can be safely encapsulated in a gelatine or gelatine based shell if the filling comprises at least one component constituent in the form of colloidal silica or silicon dioxide. The silica component included in the capsule filling is intended to fix the water component of the said mixture, in order to avoid its migration towards the capsule envelope. The result is a useful soft gelatine capsule with a filling which contains a proportion of water which is greater than has been previously possible. The colloidal silica, or silicon dioxide, may be a pharmaceutical, food, dietetic, cosmetic and/or veterinary grade silica, depending upon the intended use of the product.

The amount of colloidal silica in fillings of capsules according to the invention in relation to the total weight of a capsule is preferably in the range 0.5% to 10%.

The required levels of the colloidal silica or silicon dioxide in the capsule filling to preserve capsule stability can be influenced by the composition of the capsule shell in which the filling is confined. Particularly, we have found that by including an additional component such as starch in the shell formulation, lower levels of silica are required in the filling. Reference is directed to EP-A-0 233 231; EP-A-0 337 509 and EP-A-0 559 827 for discussion of capsule shell formulations with starch additives or substitutes. Typical starch levels in the shell formulations of capsules according to the invention are in the range 10 to 25% by weight. A suitable starch is corn starch, but others such as that available under the Trade Mark PERFECTAMYL from Avebe BA, might be used.

The invention will now be further described in the following examples:

### Example 1

A soft gelatine capsule for food or nutritional use has, for a volume of approximately 0.6 ml, a filling having the following composition:

| | |
|---|---|
| soya oil | 330 mg |
| hydrogenated soya oil | 33 mg |
| aqueous extract of garlic | 200 mg |
| soya lecithin | 20 mg |
| colloidal silica | 17 mg |

In this mixture the proportion of water is 33%, that is to say a quantity of water which would normally lead to excessive liquefaction of the gelatine shell.

The above composition is characterised by a content of colloidal silica of 17 mg which, in relation to the total weight of a capsule with a content of 600 mg, whose gelatine, glycerine and water envelope weighs 250 mg, that is to say a total weight of 850 mg, is 2%. Such a content is permitted by current regulations covering products in the category of additives and technological aids used as antiagglomerants, in accordance with EEC directive E 551 (source: DEHOVE "Product Regulation", ED LAMY, 2/1990, paragraph 6-190).

The incorporation of the colloidal silica in this aqueous formulation allows the water to be retained preventing it from being dispersed in the gelatine forming the soft capsule shell.

The colloidal silica used is perfectly suited to use as a food, pharmaceutical, dietetic, cosmetic and/or veterinary additive due to the fact that it is available in the various grades required and authorised.

In a particular test, the above filling was encapsulated in shells having the following formulation by weight:

| | |
|---|---|
| Gelatin | 30.64% |
| Glycerol | 18.96% |
| Starch | 15.00% |
| Purified Water | 35.40% |

The capsules were the subject of a stability study, being stored in glass bottles for three months at 37°C and 45°C, and for twelve months at room temperature. In each of the three month tests the capsules retained good stability apart from some darkening and at 45°C some stickiness developed in the shells. In the twelve month test good stability was also retained, with no deformation or softening of the capsule shells. In a disintegration test, after three months storage at 37°C, the capsules opened easily within five minutes after gentle shaking in water at 37°C.

### Example 2

A soft gelatine capsule for food product use has, for a volume of approximately 0.675 ml, a filling having the following composition:

| | |
|---|---|
| wheatgerm oil | 499.500 mg |
| fresh royal jelly | 150.000 mg |
| colloidal silica | 25.500 mg |

Due to the fact that the royal jelly contains an average of 66% water, the content of the capsule obtained in this way has a water content of 14.66%.

The above filling was encapsulated in shells having the following formulation by weight:

| | |
|---|---|
| Gelatin | 46.00% |
| Glycerol 100% | 17.82% |
| Purified Water | 36.18% |

The capsules were the subject of a stability study, being stored in glass bottles for three months at 37°C and 45°C, and for twelve months at room temperature. In each of the three month tests the capsules retained good stability apart from some darkening of colour and slight softening of the shells. In the twelve month test good stability was also retained, with good shape and normal hardness. The same disintegration was conducted as with the capsules described in example 1, with similar results. The capsules opened easily within 5 minutes after gentle shaking in water at 37°C.

## Claims

1. A soft gelatine capsule comprising a shell containing a filling with a high water content, characterised in that the filling includes at least one constituent in the form of colloidal silica or silicon dioxide.

2. A capsule according to Claim 1 wherein the colloidal silica, or silicon dioxide, is a silica of a pharmaceutical, food, dietetic, cosmetic and/or veterinary grade.

3. A capsule according to Claim 1 or Claim 2 wherein the filling includes colloidal silica in an amount in relation to the total weight of a capsule in the range 0.5% to 10%.

4. A capsule according to any preceding Claim wherein the capsule shell formulation includes a starch.

5. A capsule according to Claim 4 wherein the amount of starch in the shell formulation is in the range 10 to 25% by weight.

6. A capsule according to any preceding Claim wherein the amount by weight of colloidal silica or silicon dioxide in the filling does not exceed 2% of the total capsule.

7. A capsule according to any preceding Claim wherein, for a volume of substantially 0.60 ml, the filling has the following composition:
| | |
|---|---|
| soya oil | 330 mg |
| hydrogenated soya oil | 33 mg |
| aqueous extract of garlic | 220 mg |
| soya lecithin | 20 mg |
| colloidal silica | 17 mg |

8. A capsule according to Claim 7 wherein the capsule shell has the following formulation by weight:
| | |
|---|---|
| Gelatin | 30.64% |
| Glycerol | 18.96% |
| Starch | 15.00% |
| Purified Water | 35.40% |

9. A capsule according to any of Claims 1 to 3 wherein, for a volume of approximately 0.675 ml, the filling has the following composition:
| | |
|---|---|
| wheatgerm oil | 499.500 mg |
| fresh royal jelly | 150.000 mg |
| colloidal silica | 25.500 mg |

10. A capsule according to Claim 9 wherein the capsule shell has the following formulation by weight:
| | |
|---|---|
| Gelatin | 46.00% |
| Glycerol 100% | 17.82% |
| Purified Water | 36.18% |
